# EUROPÄISCHE PATENTANMELDUNG

(11) **EP 2 020 206 A1**
(43) Veröffentlichungstag der Anmeldung: **04.02.2009**
(21) Anmeldenummer: 08400037.1
(22) Anmeldetag: 26.07.2008
(51) Int. Cl.: A61B 3/12, G06K 9/00

(54) **Verfahren und Anordnung zur automatischen Erkennung und Interpretation der Irisstruktur als eine Zustandsbestimmung einer Person**

(30) Priorität: 28.07.2007 DE 102007035873
(71) Anmelder: Perner, Petra, 04275 Leipzig (DE)
(72) Erfinder: Perner, Petra, 04275 Leipzig (DE)
(74) Vertreter: Krause, Wolfgang

(57) **Zusammenfassung**

Bei Verfahren und Anordnungen zur automatischen Erkennung und Interpretation der Irisstruktur als eine Zustandsbestimmung einer Person ist der Zustand der Person einfach bestimmbar.

Dazu erfolgt mit entsprechenden Einrichtungen eines Datenverarbeitungssystems
- eine zweidimensionale Aufnahme und Digitalisierung eines Bereiches eines Augapfels mit der Iris mittels einer mit einem Datenverarbeitungssystem verbundenen Aufnahmevorrichtung,
- eine Auswahl der Iris,
- eine Erstellung der Topographie der Iris durch Aufteilung des Bildes der Iris in Bilder von Kreisringabschnitten,
- eine Ermittlung von Merkmalen für die Kreisringabschnitte und/oder von Objekten in den Bildern der Kreisringabschnitte durch Zusammenfassen von Bildpunkten gleicher Farbe oder Intensität zu einzelnen Objekten mit einem Objektisolierungsverfahren,
- eine Merkmalsbestimmung der durch das Objektisolierungsverfahren ermittelten Objekte,
- ein Vergleich der bestimmten Merkmale der Objekte mit als Klassifikatorwissen im Datenverarbeitungssystem gespeicherten Merkmalen und
- eine Zuordnung der bestimmten Objekte zu Körperregionen entsprechend der Topographie der Iris.

## Beschreibung

Die Erfindung betrifft Verfahren und Anordnungen zur automatischen Erkennung und Interpretation der Irisstruktur als eine Zustandsbestimmung einer Person.

Durch die Druckschrift DE 20 2006 012 628 U1 (Vorrichtung zur Ermittlung medizinischer und/oder biometrischer Daten eines Lebewesns) ist eine Vorrichtung zur Erfassung, zur Speicherung, zum Abgleich und zur Auswertung von medizinischen Merkmalen eines Lebewesens und/oder von die Einzigartigkeit und damit die Identität eines Lebewesens bestimmenden biometrischen Merkmalen bekannt. Diese Lösung betrifft die Aufnahme, das Übertragen und das sichere Speichern derartiger Daten. Eine Auswertung bestimmter Merkmale der Bilder der Aufnahmen ist nicht vorgesehen. Die Druckschrift DE 10 2005 008 399 A1 (System der Distanz-Uninvasionsdiagnostik) beinhaltet ein System der Distanz-Uninvasionsdiagnostik, wobei mittels dem optischen Zustand und einer digitalen Fotokamera eine Übertragung des Bildes der Regenbogenhaut für die Feststellung des Gesundheitszustandes des Menschen mit Hilfe von einer automatisierten Methode der Irisdiagnostik verwirklicht wird. Das Bild der aufgenommenen Regenbogenhaut wird an einem Computer zur Diagnostik übertragen. Die Diagnostik selbst erfolgt über das dargestellte Bild manuell.

Der in den Patentansprüchen 1, 6, 8 und 9 angegebenen Erfindung liegt das Problem zugrunde, den Zustand einer Person über ein Erkennen und Interpretieren der Irisstruktur einfach zu bestimmen.

Dieses Problem wird mit den in den Patentansprüchen 1, 6, 8 und 9 aufgeführten Merkmalen gelöst.

Die Verfahren und Anordnungen zur automatischen Erkennung und Interpretation der Irisstruktur als eine Zustandsbestimmung einer Person zeichnen sich insbesondere aus, das der Zustand der Person einfach bestimmbar ist. Dazu erfolgt
- eine zweidimensionale Aufnahme und Digitalisierung eines Bereiches eines Augapfels mit der Iris mittels einer mit einem Datenverarbeitungssystem verbundenen Aufnahmevorrichtung,
- eine Auswahl der Iris,
- eine Erstellung der Topographie der Iris durch Aufteilung des Bildes der Iris in Bilder von Kreisringabschnitten,
- eine Ermittlung von Merkmalen für die Kreisringabschnitte und/oder von Objekten in den Bildern der Kreisringabschnitte durch Zusammenfassen von Bildpunkten gleicher Farbe oder Intensität zu einzelnen Objekten mit einem Objektisolierungsverfahren,
- eine Merkmalsbestimmung der durch das Objektisolierungsverfahren ermittelten Objekte,
- ein Vergleich der bestimmten Merkmale der Objekte mit als Klassifikatorwissen im Datenverarbeitungssystem gespeicherten Merkmalen und
- eine Zuordnung der bestimmten Objekte zu Körperregionen entsprechend der Topographie der Iris.

Darüber hinaus ist bei der Anordnung zur automatischen Erkennung und Interpretation der Irisstruktur als eine Zustandsbestimmung einer Person entweder eine einen Bereich des Augapfels mit der Iris zweidimensional erfassende und digitalisierende Aufnahmevorrichtung oder ein Datenspeicher mit digitalisierten Irisaufnahmen mit mindestens einem das digitalisierte Bild farbecht oder als gewandeltes Grauwertbild aufnehmenden Datenverarbeitungssystem zusammengeschaltet. Im Datenverarbeitungssystem sind weiterhin
- eine die Iris als Kreisringfläche auswählende,
- eine die Topographie der Iris durch Aufteilung des Bildes der Iris in Bilder von Kreisringabschnitten erstellende,
- eine Merkmale für die Kreisringabschnitte und/oder Objekte in den Bildern der Kreisringabschnitte durch Zusammenfassen von Bildpunkten gleicher Farbe oder Intensität zu einzelnen Objekten mit einem Objektisolierungsverfahren ermittelnde,
- eine Merkmale der durch das Objektisolierungsverfahren ermittelten Objekte bestimmende,
- eine die bestimmten Merkmale der Objekte mit als Klassifikatorwissen im Datenverarbeitungssystem gespeicherten Merkmalen vergleichende und
- eine die bestimmten Objekten zu Körperregionen entsprechend der Topographie der Iris zuordnende Einrichtung
   realisiert und nacheinander verknüpft.

Die Iris ist die durch Pigmente gefärbte Blende des Auges und befindet sich am Übergang von der Hornhaut zur Sclera. Die Iris reguliert den Lichteinfall in das Auge. Die Iris schließt mit der Iriswurzel am Zeliarkörper an und lässt in ihrer Mitte eine Öffnung, die Pupille frei, frei. Die Iris besteht aus zwei Schichten. Die vordere Schicht ist das Stroma, das bei jedem Menschen unterschiedlich ausgeprägt ist. Ein hoher Pigmentanteil im Stroma färbt die Iris braun, ein niedrigerer grün bis blau oder grau. Die hintere Schicht ist das Pigmentblatt. Das im Pigmentblatt enthaltene Pigment bewirkt ein Filtern des Streulichts.

Über die Iris können mittels der Iridologie, auch Irisanalyse oder Irisdiagnostik, die Konstitution, die Disposition für Krankheiten und Krankheiten selbst erkannt werden. Dazu sind Bereiche der Iris einzelnen Organen und Körperteilen topographisch zugeordnet. Mittels der Struktur der Iris und der Zuordnung zu den Bereichen der Iris kann auf die Konstitution, die Disposition für Krankheiten und Krankheiten geschlossen werden.

Gegenstand der Iridologie oder auch Irisdiagnostik ist, dass durch Beobachtung des Zustandes und der Veränderlichkeit des sichtbaren Irisgewebes Aussagen über Erkrankungen oder Prädispositionen des Körpers gemacht werden könnten. Grundlage ist, dass von der gesamten Peripherie mit allen Organen Leitungsbahnen zur Iris führen. Dazu sind topographische Übersichten der Iris aus der Iridologie und der klinischen Prüfung der Iriszeichen bekannt.

Vorteilhafterweise erfolgt das mittels des Verfahrens und der Anordnung nach der Aufnahme der Iris automatisch.

Vorteilhafterweise wird das weiterhin durch ein Computer-Programm mit einem Programmcode entweder zur Durchführung des Verfahrens zur automatischen Erkennung und Interpretation der Irisstruktur als eine Zustandsbestimmung einer Person, wenn das Programm auf einem Rechner abläuft,
und durch ein Computer-Programm-Produkt auf einem maschinenlesbaren Träger entweder zur Durchführung des Verfahrens zur automatischen Erkennung und Interpretation der Irisstruktur als eine Zustandsbestimmung einer Person, wenn das Programm auf einem Rechner abläuft, erreicht.

Vorteilhafterweise sind sowohl
- ein Computer-Programm mit einem Programmcode zur Durchführung des Verfahrens zur automatischen Erkennung und Interpretation der Irisstruktur als eine Zustandsbestimmung einer Person, wenn das Programm auf einem Rechner abläuft, und
- ein Computer-Programm-Produkt auf einem maschinenlesbaren Träger zur Durchführung des Verfahrens zur automatischen Erkennung und Interpretation der Irisstruktur als eine Zustandsbestimmung einer Person, wenn das Programm auf einem Rechner abläuft,
   eingeschlossen.
   Vorteilhafte Ausgestaltungen der Erfindung sind in den Patentansprüchen 2 bis 5 und 7 angegeben.
   Die Iris als Iriskrause in Form einer Kreisringfläche wird nach der Weiterbildung des Patentanspruchs 2 aus dem digitalisierten Bild über eine Kreismaske für die Eliminierung der Pupille und eine Lochmaske für die Außenkontur der Iris ausgewählt und bestimmt.
   Nach der Weiterbildung des Patentanspruchs 3 wird das bestimmte Objekt im Bild der Iris in einem mit dem Datenverarbeitungssystem verbundenen Bildschirm dargestellt und/oder mit seiner Position in der Iris im Speicher des Datenverarbeitungssystems abgelegt. Dadurch wird auch eine manuelle Auswertung vereinfacht.
   Nach der Weiterbildung des Patentanspruchs 4 wird vorteilhaftereise wenigstens eines der Merkmale
- Objekt im Bild eines Kreisringabschnittes vorhanden oder nicht,
- Anzahl von Objekten,
- Fläche des Objektes,
- mittlere Fläche des Objektes,
- relative mittlere Fläche des Objektes,
- mittlerer Formfaktor,
- mittlere Konturlänge,
- Position des Objektes im Bild des Kreisringabschnittes,
- Farbe oder Grauwert des Objektes und
- Textur
dem bestimmten Objekt zugeordnet.

Nach der Weiterbildung des Patentanspruchs 5 wird
- ein unbekanntes oder unsicher klassifiziertes Objekt automatisch erkannt,
- das Objekt auf einem mit dem Datenverarbeitungssystem verbundenen Datensichtgerät dargestellt und
- dieses Merkmal über eine Lerneinheit automatisch dem Klassifikatorwissen zu- und eingeordnet.

Dazu ist nach der Weiterbildung des Patentanspruchs 7 eine ein unbekanntes oder unsicher klassifiziertes Objekt und daraus ein bestimmtes neues Merkmal automatisch dem Klassifikatorwissen zuordnende Lerneinheit mit den die Merkmale für die einzelnen Objekte vergleichenden Einrichtung zusammengeschaltet.

Die Lerneinheit gestattet es damit, unbekannte Merkmale der Objekte durch eine Bewertung des Nutzers und/oder Bedienpersonals aufzunehmen und automatisch in die im Datenverarbeitungssystem als Klassifikatorwissen vorhandenen, gespeicherten und bekannten Merkmalen ein- und zuzuordnen.

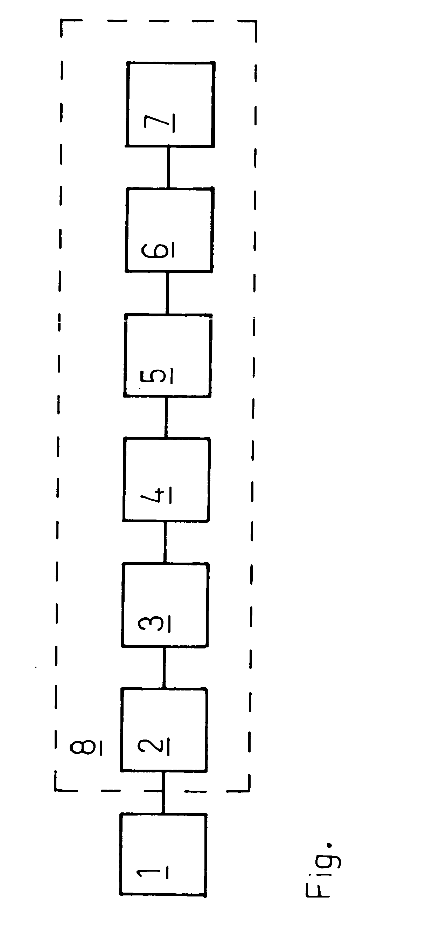

Ein Ausführungsbeispiel der Erfindung ist in der Zeichnung prinzipiell dargestellt und wird im folgenden näher beschrieben.

Es zeigt die
Fig. Eine Anordnung zur automatischen Erkennung und Interpretation der Irisstruktur als eine Zustandsbestimmung einer Person.

Im nachfolgenden Ausführungsbeispiel werden ein Verfahren und eine Anordnung zur automatischen Erkennung und Interpretation der Irisstruktur als eine Zustandsbestimmung einer Person zusammen näher beschrieben.

Die Anordnung besteht im Wesentlichen aus entweder einer einen Bereich des Augapfels mit der Iris zweidimensional erfassenden und digitalisierenden Aufnahmevorrichtung 1 oder einem Datenspeicher mit Irisaufnahmen sowie mindestens einem das digitalisierte Bild farbecht oder als gewandeltes Grauwertbild aufnehmenden Datenverarbeitungssystem 8. Im Datenverarbeitungssystem 8 sind
- eine die Iris als Kreisringfläche auswählende Einrichtung 2,
- eine die Topographie der Iris durch Aufteilung des Bildes der Iris in Bilder von Kreisringabschnitten erstellende Einrichtung 3,
- eine Merkmale für die Kreisringabschnitte und/oder Objekte in den Bildern der Kreisringabschnitte durch Zusammenfassen von Bildpunkten gleicher Farbe oder Intensität zu einzelnen Objekten mit einem Objektisolierungsverfahren ermittelnde Einrichtung 4,
- eine Merkmale der durch das Objektisolierungsverfahren ermittelten Objekte bestimmende Einrichtung 5,
- eine die bestimmten Merkmale der Objekte mit als Klassifikatorwissen im Datenverarbeitungssystem gespeicherten Merkmalen vergleichende Einrichtung 6 und
- eine die bestimmten Objekten zu Körperregionen entsprechend der Topographie der Iris zuordnende Einrichtung 7 realisiert.

Die Fig. zeigt eine Anordnung zur automatischen Erkennung und Interpretation der Irisstruktur als eine Zustandsbestimmung einer Person in einer prinzipiellen Darstellung.

Mittels der Aufnahmevorrichtung 1 erfolgt eine zweidimensionale Aufnahme und Digitalisierung eines Bereiches eines Augapfels mit der Iris. Das ist vorzugsweise eine Digitalkamera, wobei das Bild vorteilhafterweise über ein Mikroskop aufgenommen wird. Die Aufnahmevorrichtung 1 ist mit dem Datenverarbeitungssystem 8 verbunden.

Natürlich kann auch ein Datenspeicher mit Irisaufnahmen mit dem Datenverarbeitungssystem 8 verbunden sein. Dadurch ist eine örtliche Trennung zwischen Aufnahmevorrichtung 1 und Datenverarbeitungssystem 8 vorhanden.
Im Datenverarbeitungssystem 8 sind die Einrichtungen 2 bis 7 nacheinander verknüpft und zusammengeschaltet.

In einer die Iris als Kreisringfläche auswählende Einrichtung 2 wird die Iris als Iriskrause in Form einer Kreisringfläche aus dem digitalisierten Bild über eine Kreismaske für die Eliminierung der Pupille und eine Lochmaske für die Außenkontur der Iris ausgewählt und bestimmt.

Die Topographie der Iris wird in der Einrichtung 3 durch Aufteilung des Bildes der Iris in Bilder von Kreisringabschnitten erstellt. Diese Kreisringabschnitte sind bestimmten Körperteilen zugeordnet.

Danach erfolgt eine Ermittlung der Objekte in den Bildern der Kreisringabschnitte. Dazu werden Bildpunkten gleicher Farbe oder Intensität zu einzelnen Objekten zusammengefasst und mit einem Objektisolierungsverfahren ermittelt. Das erfolgt in der dafür vorgesehenen Einrichtung 4.

Merkmale der durch das Objektisolierungsverfahren ermittelten Objekte werden in der Einrichtung 5 bestimmt. Dabei wird wenigstens eines der Merkmale
- Objekt im Bild eines Kreisringabschnittes vorhanden oder nicht,
- Anzahl von Objekten,
- Fläche des Objektes,
- mittlere Fläche des Objektes,
- relative mittlere Fläche des Objektes,
- mittlerer Formfaktor,
- mittlere Konturlänge,
- Position des Objektes im Bild des Kreisringabschnittes,
- Farbe oder Grauwert des Objektes und
- Textur
dem bestimmten Objekt zugeordnet.

Die bestimmten Merkmale der Objekte werden mit als Klassifikatorwissen im Datenverarbeitungssystem 8 mit gespeicherten Merkmalen in der dafür vorgesehenen Einrichtung 6 verglichen.

Abschließend werden die bestimmten Objekten zu Körperregionen entsprechend der Topographie der Iris in der Einrichtung 7 zugeordnet.

Das bestimmte Objekt wird im Bild der Iris in einem mit dem Datenverarbeitungssystem 8 verbundenen Bildschirm dargestellt und/oder mit seiner Position in der Iris im Speicher des Datenverarbeitungssystems 8 abgelegt.

Ein unbekanntes oder unsicher klassifiziertes Objekt wird darüber hinaus automatisch erkannt, das Objekt auf einem mit dem Datenverarbeitungssystem 8 verbundenen Datensichtgerät dargestellt und dieses Merkmal über eine Lerneinheit automatisch dem Klassifikatorwissen zu- und eingeordnet.
Dazu ist eine ein unbekanntes oder unsicher klassifiziertes Objekt und daraus ein bestimmtes neues Merkmal automatisch dem Klassifikatorwissen zuordnende Lerneinheit mit den die Merkmale für die einzelnen Objekte vergleichenden Einrichtung zusammengeschaltet.

Das Ausführungsbeispiel kann darüber hinaus
- ein Computer-Programm mit einem Programmcode zur Durchführung des Verfahrens zur automatischen Erkennung und Interpretation der Irisstruktur als eine Zustandsbestimmung einer Person, wenn das Programm auf einem Rechner abläuft, und
- ein Computer-Programm-Produkt auf einem maschinenlesbaren Träger zur Durchführung des Verfahrens zur automatischen Erkennung und Interpretation der Irisstruktur als eine Zustandsbestimmung einer Person, wenn das Programm auf einem Rechner abläuft,
beinhalten.

## Patentansprüche

1. Verfahren zur automatischen Erkennung und Interpretation der Irisstruktur als eine Zustandsbestimmung einer Person mit folgenden Schritten:
- zweidimensionale Aufnahme und Digitalisierung eines Bereiches eines Augapfels mit der Iris mittels einer mit einem Datenverarbeitungssystem (8) verbundenen Aufnahmevorrichtung (1),
- Auswahl der Iris,
- Erstellung der Topographie der Iris durch Aufteilung des Bildes der Iris in Bilder von Kreisringabschnitten,
- Ermittlung von Merkmalen für die Kreisringabschnitte und/oder von Objekten in den Bildern der Kreisringabschnitte durch Zusammenfassen von Bildpunkten gleicher Farbe oder Intensität zu einzelnen Objekten mit einem Objektisolierungsverfahren,
- Merkmalsbestimmung der durch das Objektisolierungsverfahren ermittelten Objekte,
- Vergleich der bestimmten Merkmale der Objekte mit als Klassifikatorwissen im Datenverarbeitungssystem gespeicherten Merkmalen und
- Zuordnung der bestimmten Objekte zu Körperregionen entsprechend der Topographie der Iris.

2. Verfahren nach Patentanspruch 1, **dadurch gekennzeichnet, dass** die Iris als Iriskrause in Form einer Kreisringfläche aus dem digitalisierten Bild über eine Kreismaske für die Eliminierung der Pupille und eine Lochmaske für die Außenkontur der Iris ausgewählt und bestimmt wird.

3. Verfahren nach Patentanspruch 1, **dadurch gekennzeichnet, dass** das bestimmte Objekt im Bild der Iris in einem mit dem Datenverarbeitungssystem verbundenen Bildschirm dargestellt und/oder dass das bestimmte Objekt mit seiner Position in der Iris im Speicher des Datenverarbeitungssystems (8) abgelegt wird.

4. Verfahren nach Patentanspruch 1, **dadurch gekennzeichnet, dass** wenigstens eines der Merkmale
- Objekt im Bild eines Kreisringabschnittes vorhanden oder nicht,
- Anzahl von Objekten,
- Fläche des Objektes,
- mittlere Fläche des Objektes,
- relative mittlere Fläche des Objektes,
- mittlerer Formfaktor,
- mittlere Konturlänge,
- Position des Objektes im Bild des Kreisringabschnittes,
- Farbe oder Grauwert des Objektes und
- Textur
dem bestimmten Objekt zugeordnet wird.

5. Verfahren nach Patentanspruch 1, **dadurch gekennzeichnet, dass** ein unbekanntes oder unsicher klassifiziertes Objekt automatisch erkannt, dass das Objekt auf einem mit dem Datenverarbeitungssystem (8) verbundenen Datensichtgerät dargestellt und dass dieses Merkmal über eine Lerneinheit automatisch dem Klassifikatorwissen zu- und eingeordnet wird.

6. Anordnung zur automatischen Erkennung und Interpretation der Irisstruktur als eine Zustandsbestimmung einer Person, **dadurch gekennzeichnet,**
**dass** entweder eine einen Bereich des Augapfels mit der Iris zweidimensional erfassende und digitalisierende Aufnahmevorrichtung (1) oder ein Datenspeicher mit digitalisierten Irisaufnahmen mit mindestens einem das digitalisierte Bild farbecht oder als gewandeltes Grauwertbild aufnehmenden Datenverarbeitungssystem (8) zusammengeschaltet ist und dass im Datenverarbeitungssystem (8)
- eine die Iris als Kreisringfläche auswählende (2),
- eine die Topographie der Iris durch Aufteilung des Bildes der Iris in Bilder von Kreisringabschnitten erstellende (3),
- eine Merkmale für die Kreisringabschnitte und/oder Objekte in den Bildern der Kreisringabschnitte durch Zusammenfassen von Bildpunkten gleicher Farbe oder Intensität zu einzelnen Objekten mit einem Objektisolierungsverfahren ermittelnde (4),
- eine Merkmale der durch das Objektisolierungsverfahren ermittelten Objekte bestimmende (5),
- eine die bestimmten Merkmale der Objekte mit als Klassifikatorwissen im Datenverarbeitungssystem gespeicherten Merkmalen vergleichende (6) und
- eine die bestimmten Objekten zu Körperregionen entsprechend der Topographie der Iris zuordnende (7)
Einrichtung
realisiert sind und nacheinander verknüpft sind.

7. Anordnung nach Patentanspruch 6, **dadurch gekennzeichnet, dass** eine ein unbekanntes oder unsicher klassifiziertes Objekt und daraus ein bestimmtes neues Merkmal automatisch dem Klassifikatorwissen zuordnende Lerneinheit mit den die Merkmale für die einzelnen Objekte vergleichenden Einrichtung (6) zusammengeschaltet ist.

8. Computer-Programm mit einem Programmcode zur Durchführung des Verfahrens zur automatischen Erkennung und Interpretation der Irisstruktur als eine Zustandsbestimmung einer Person nach Anspruch 1, wenn das Programm auf einem Rechner abläuft.

9. Computer-Programm-Produkt auf einem maschinenlesbaren Träger zur Durchführung des Verfahrens zur automatischen Erkennung und Interpretation der Irisstruktur als eine Zustandsbestimmung einer Person nach Anspruch 1, wenn das Programm auf einem Rechner abläuft.
